# EUROPEAN PATENT SPECIFICATION

(11) **EP 1 363 892 B1**
(45) Date of publication and mention of the grant of the patent: **16.08.2006**
(21) Application number: 01981204.9
(22) Date of filing: 19.10.2001
(51) Int. Cl.: C07D 305/14, A61K 31/337, A61P 35/00

(54) **METHOD FOR THE PRODUCTION OF A STABLE PHARMACEUTICAL FORM OF PACLITAXEL**
VERFAHREN ZUR HERSTELLUNG EINER BESTÄNDIGE PHARMAZEUTISCHE FORM VON PACLITAXEL
PROCEDE PERMETTANT LA PRODUCTION D'UNE FORME PHARMACEUTIQUE STABLE DE PACLITAXEL

(30) Priority: 31.10.2000 PL 34361500
(43) Date of publication of application: 26.11.2003
(73) Proprietor: Warszawskie Zaklady Farmaceutyczne Polfa Spolka Akcyjna, 01-207 Warszawa (PL); Instytut Chemii Organicznej Pan, 01-244 Warszawa (PL); Akademia Medyczna, 80-416 Gdansk (PL)
(72) Inventor: JANICKI, Stanislaw, DECEASED (PL); SZNITOWSKA, Malgorzata, PL-80-772 Gdansk (PL); KARAS, Wladyslaw, PL-03-580 Warszawa (PL); MROCZEK, Zdzislaw, PL-01-315 Warszawa (PL); TYRALA, Andrzej, PL-01-493 Warszawa (PL); CZARNECKA, Iwona, PL-03-133 Warszawa (PL)
(74) Representative: Schwabe - Sandmair - Marx
(86) International application number: PCT/PL2001/000082
(87) International publication number: WO 2002/036582

(56) References cited:
- EP-A- 0 717 041
- CHEMICAL ABSTRACTS, vol. 136, no. 1, 2002 Columbus, Ohio, US; abstract no. 11026r, LEE,J.H.: "PREPARATION A. CHARACTERISATION OF SOLVENT INDUCED DIHYDRATE,ANHYDROUS A.AMORPHOUS PACLITAXEL." page 1219; XP002190101 & BULLETIN OF THE KOREAN CHEMICAL SOCIETY, vol. 22, no. 8, 2001, pages 925-8, SOUTH-KOREA
- LIGGINS R.T. ET AL: 'Solid-State Characterization of Paclitaxel' J.PHARM.SCI. vol. 86, no. 12, 1997, pages 1458 - 1463, XP009027537

## Description

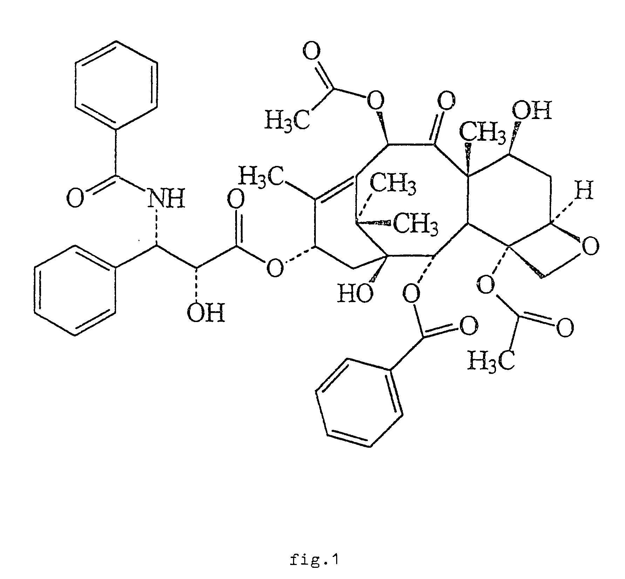

The Present invention concerns a method for production of a stable pharmaceutical form of an anticancer drug containing exclusively, or in addition to other active substances, the chemical compound insoluble in water named paclitaxel, presented by the Fig. 1.

Paclitaxel of the Fig. 1 is a known chemical compound isolated from the bark of the tree Taxus brevifolia or obtained through chemical synthesis. This compound is used in medicine in the treatment of malignant neoplasms resistant, to other methods of therapy (Slichenmyer WJ, Von Hoff DD. New natural products in cancer chemotherapy. J. Clin Pharmacol 1990; 3.0: 770-88, Rowinrisky EKy et al. Taxol: a novel investigational anti microtubule agent, J. Natl. Cancer Inst. 1990; 82: 1247-59, Long HJ, Paclitaxel (Taxol): a novel anticancer chemotherapeutic drug Mayo Clin. Proc. 1994; 69: Donehower RC. Paclitaxel (Taxol). N.Engl. J. Med. 1995, 332: and other papers).

In cancer therapy, paclitaxel is administered intravenously, thus it is necessary to make solutions of appropriate concentration in biocompatible solvent systems.

Very slight solubility of paclitaxel in water makes impossible its direct administration in classical, commonly used infusion fluids. Therefore, the only hitherto used commercial forms of pharmaceuticals containing paclitaxel are the "basic" solutions of this substance in a mixture of a polar solvent and a surface-active substance, which cannot be injected to a patient directly, but only after prior dilution with an infusion fluid.

The best-known double-solvent system used for making the "basic" solutions of pacitaxel is a mixture of polyethoxylated castor oil, known under the trade name of Cremophor EL, and ethyl alcohol. The characteristic feature of those solutions is that after being diluted with an infusion fluid they can precipitate. Moreover, chemical stability of paclitaxel in such solutions is low. Already after 2 months of storage at the temperature of 25°C, 10% reduction in the active substance content was observed, and after 22 months the content of paclitaxel was only 67% of its initial quantity. The rate of decomposition at elevated temperatures was considerably higher, Instability of paclitaxel in such solutions is connected with the chemical properties of Crem.ophor EL and the presence of even a small quantity of water in ethyl alcohol. It is obvious that such a preparation has very limited applications and its administration to a patient involves a real risk consequential to the administration of too low a dose of the drug due to its decomposition.

Richard T. Liggins et al. (J. Pharm. Sci. 86, p. 1458 [1997]) characterized the solid-state properties of anhydrous paclitaxel ("paclitaxel I"), paclitaxel dihydrate. The samples were analyzed by differential scanning calorimetry (DSC), thermogravimetric analysis (TGA) and X-ray powder diffraction. DSC of paclitaxel dihydrate resulted in completely dehydrated paclitaxel ("dehydrated paclitaxel·2H₂O") which in turn was transformed at higher temperatures into a semicrystalline material ("paclitaxel I/am"). X-ray powder diffraction confirmed that paclitaxel I, paclitaxel dihydrate and dehydrated paclitaxel I/am did not behave identically when analyzed by DSC. Bulk dissolution studies demonstrated the existence of a dihydrate form of paclitaxel that is the stable form in equilibrium with water at 37 °C but which dehydrates at temperatures > 45 °C.

In patent no. PL 176826, a procedure partially eliminating the above-mentioned disadvantages was proposed. It consisted in complex commercial purification of Cremophor EL so as to eliminate those chemical properties of paclitaxel that compromised its stability, or in addition of an acid so as to reduce the solvent concentration of carboxylic anions affecting the substance stability, or in utilisation of anhydrous ethyl alcohol. The solutions obtained in that way were significantly more stable and they could be stored for a longer period of time at room temperature without an evident loss of activity. However, after those solutions are diluted with an infusion fluid there is a risk of precipitation, and that is why it is necessary to use an : infusion set provided with an appropriate filter.

However, the above procedure does not eliminate a serious disadvantage of thus produced pharmaceutical, connected with the application of Cremophor EL whose presence in medicinal products administered intravenously may in some cases result in the risk of anaphylactic shock (Rowinnsky EK, et. al. Taxol: a novel investigational antimicrotubule agent. J. Natl.. Cancer Inst. 1990; 82: 1247-59).

Furthermore, the presence of ethyl alcohol in a medicinal product when dosage regimens requiring administration of large quantities of the drug are applied may lead to intoxication symptoms.

In the patent description PL 169372, the method was presented for production of paclitaxel solutions of higher concentrations of the active substance, which reduces the harmful action of Cremophor EL. The method also allows to eliminate, nearly completely ethyl alcohol from the preparation, and thus to reduce considerably the harmful action of that second component of the drug.

Unexpectedly, it turned out that the application of the new pharmaceutical form obtained by the method according to the invention solved all the above-mentioned problems with the stability of the paclitaxel-containing medicine and allowed to eliminate some harmful components used up to now.

According to the invention, the method for production of the stable pharmaceutical form of the anticancer drug consists in dissolution of crystalline paclitaxel in a neutral organic solvent selected from the group comprising acetonitrile, dioxane, ethanol, or a mixture of those solvents, with possible' addition of water, providing that the content of individual solvents in the- mixture is in the range of 5% to 95%, while the content of water is in the range- of 0 to 60%; the resulting solution is possibly filtered, frozen, and solvents are removed by sublimation under reduced pressure, at a low temperature, and thereafter the preparation is possibly distributed into doses in conditions assuring its sterility.

Preferably, in the method according to the invention, the possibly filtered solution of paciltaxel is first dosed into containers, and then it is frozen, and solvents are removed by sublimation under reduced pressure, at a low temperature.

In the method according to the invention, solvent removal by sublimation is preferably performed at the temperature ranging from -60°C to +50°C.

The pharmaceutical form of the anticancer drug of the method according to the invention is very stable chemically and at the same time freely soluble in various biocompatible biocompatible solvents systems, which makes possible its administration to the patient.

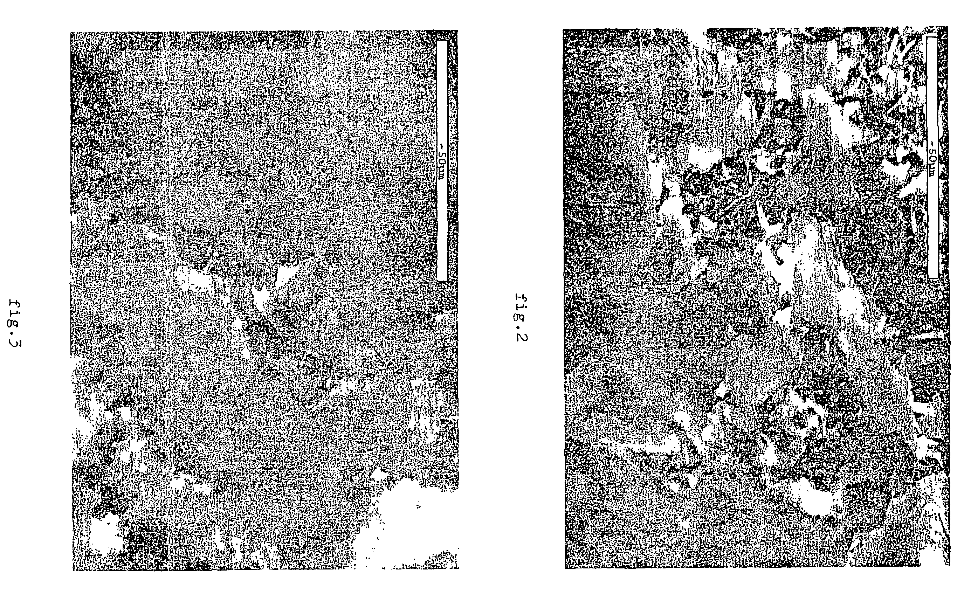

Paclitaxel isolated from Taxus brevifolia or obtained through chemical syntheses is a crystalline substance as presented in Fig.2, whose heat of dissolution in ethyl alcohol is 66.6.J/g.

Unexpectedly, it turned out that the substance prepared by the method according to the invention was a form where the amorphous structure as presented in Fig. 3 was prevalent and whose heat of dissolution in ethanol, equal to 32.4 J/g, was twice tower in comparison with the crystalline form.

Because the drug is dissolved immediately before its administration, there are no complications connected with the stability of thus prepared solutions, while such complications are observed in the case of the finished formulation in the form of the "basic" solutions available to date date.

Furthermore, this stable form of paclitaxel produced by the method of the invention does not limit the selection of therapeutically appropriate solvents.

The drug prepared in the above manner is stable and it can be dissolved immediately before its administration in various solvent systems containing such components as polyoxyethylene glycols, polysorbates, polyoxyethylated fatty acids; ethanol, phospholipid fractions, tecithines, oil-in-water emulsions, propylene glycol, benzyl alcohol, dimethylacetamide, methylacetamide, saccharide esters and ethers of fatty acids, ethylene oxide copolymer, propylene oxide copolymer, sodium glycocholate, and others. The obtained solutions can be administered directly or following their dilution with the appropriate infusion fluid.
Performance of the invention is presented by the following examples.

### Example 1.

1500 mg of paclitaxel were dissolved in 100 ml of 1,4-dioxane. The resulting solution was sterilised in aseptic conditions by filtering through a 0.2 µm teflon filter. Then the solution was filled at the doses of 2 ml into 20-ml sterile glass vials. The vials were provided with sterile rubber stoppers for freeze-drying. The vials were placed in a freeze dryer provided with a device for closing the vials after the end of the sublimation drying process. The solutions were frozen to the temperature of -40°C. The drying process was conducted at the pressure of 0.1 mbar with gradual increase of the plate temperature up to +30°C. After the drying process was terminated, the vials were closed and the vacuum was reduced. After the vials were taken out of the freeze drier, they were capped and provided with the corresponding labels. Each vial contained 30 mg of paclitaxel. The medicine obtained in this way contains not more than 380 ppm of dioxane and is stable for at least 3 years.

### Example II.

1500 mg of paclitaxel were dissolved in 80 ml of 1,4-dioxane. 20 ml of water were added. The resulting solution was sterilised in aseptic conditions by filtering through a 0.2 µm teflon filter. Then the solution was filled at the doses of 2 ml into 20-ml sterile glass vials. The vials were provided with sterile rubber stoppers for freeze-drying. The vials were placed in a freeze dryer provided with a device for closing the vials after the end of the sublimation drying process. The solutions were frozen to the temperature of -40°C. The drying process was conducted at the pressure of 0.1 mbar with gradual increase of the plate temperature up to +30°C. After the drying process was terminated, the vials were closed and the vacuum was reduced. After the vials were taken out of the freeze drier, they were capped and provided with the corresponding labels. Each vial contained 30 mg of paclitaxel. The medicine obtained in this way contains not more than 380 ppm of dioxane and is stable for at least 3 years.

### Example III.

1500 mg of paclitaxel were dissolved in 50 ml of 1,4-dioxane. 50 ml of water were added. The resulting solution was sterilised in aseptic conditions by filtering through a 0.2 µm teflon filter. Then the solution was filled at the doses of 2 ml into 20-ml sterile glass vials. The vials were provided with sterile rubber stoppers for freeze-drying. The vials were placed in a freeze dryer provided with a device for closing the vials after the end of the sublimation drying process. The solutions were frozen to the temperature of -40°C. The solvent sublimation process was conducted at the pressure of 0.1 mbar with gradual increase of the plate temperature up to +30°C. After the drying process was terminated, the vials were closed and the vacuum was reduced. After the vials were taken out of the freeze drier, they were capped and provided with the corresponding labels. Each vial contained 30 mg of paclitaxel. The medicine obtained in this way contains not more than 380 ppm of dioxane and is stable for at least 3 years.

### Example IV.

30 ml of ethanol were added to 40 ml of 1,4-dioxane. 1500 mg of paclitaxel were dissolved in that solvent. 30 ml of water were added. The resulting solution was sterilised in aseptic conditions by filtering through a 0.2 µm teflon filter. Then the solution was filled at the doses of 2 ml into 20-ml sterile glass vials. The vials were provided with sterile rubber stoppers for freeze-drying. The vials were placed in a freeze dryer provided with a device for closing the vials after the end of the sublimation drying process. The solutions were frozen to the temperature of -50°C. The drying process was conducted at the pressure of 0.1 mbar with gradual increase of the plate temperature up to +40°C. After the drying process was terminated, the vials were closed and the vacuum was reduced. After the vials were taken out of the freeze drier, they were capped and provided with the corresponding labels. Each vial contained 30 mg of paclitaxel. The medicine obtained in this way contains not more than 380 ppm of dioxane, 0.5% of ethanol, and is stable for at least 3 years.

### Example V.

In the same manner as in Example I, 1500 mg of paclitaxel were dissolved, respectively, in 100 ml of various solvents whose composition is presented in the table below:

| | Acetonitrile | 1,4-dioxane | Ethanol | Water |
|---|---|---|---|---|
| a | 5% | 90% | 0% | 5% |
| b | 0% | 80% | 10% | 10% |
| c | 10% | 60% | 0% | 30% |
| d | 0% | 50% | 20% | 30% |
| e | 10% | 80% | 5% | 5% |

The preparations obtained as a result of freeze-drying of the solvents from a to e had an amorphous form and were stable for 3 years.

### Example VI.

5 ml of the sterile solvent consisting of 1980 mg of anhydrous ethanol and 2635 mg of polyoxyethylated castor oil were added to the fireeze-dried substance obtained according to Examples I - V. Paclitaxel is immediately completely dissolved. The resulting solution is stable for at least 2 years at the temperature of +4°C and for at least 3 months at room temperature. This solution, which is a concentrate, is added to the appropriate infusion fluid as presented in Example VIII.

### Example VII.

5 ml of the sterile solvent consisting of 300 mg of polysorbate 80, 3400 mg of pharmacopoeial polyoxyethylene glycol 300, and 1300 mg of anhydrous ethanol were added to the freeze-dried substance obtained according to Examples I - V. Paclitaxel was immediately completely dissolved. The resulting solution is stable at room temperature for at least 8 hours. This solution, which is a concentrate, is added to the appropriate infusion fluid as presented in Example VIII.

### Example VIII.

The concentrate obtained in Examples VI and VII was diluted its intravenous administration to the patient so as to obtain the final paclitaxel concentration of 0.3 - 1.2 mg/ml. The following were used for dilution: 0.9% sodium chloride solution, 5% glucose solution, and other infusion fluids such as the Ringer solution, the mixture of 5% glucose solution with 0.9% sodium chloride solution, the mixture of 5% glucose solution with the Ringer solution, 5% sorbitol solution, 6% dextrane solution, 10% or 20% or 30% oil-in-water emulsion for parenteral nutrition. Such solutions are stable at room temperature for at least 6 hours, which makes possible their safe administration to the patient.

### Example IX.

150 mg of the freeze-dried substance obtained according to Examples I - V were added to 500 ml of 30% soybean oil emulsion for intravenous infusion. The obtained fluid was mixed carefully until paclitaxel was completely dissolved. The resulting emulsion is stable at room temperature for at least 8 hours and it can be given to a patient by the intravenous route.

## Claims

1. A method for obtaining a stable pharmaceutical, form of paclitaxel as anti-cancer drug **characterised in that** crystalline paclitaxel is dissolved in a neutral organic solvent selected from the group comprising acetonitrile, dioxane, ethanol, or a mixture of those solvents, with possible addition of water, providing that the content of several solvents in the mixture is in the range of 5% to 95%, while the content of water is in the range of 0 to 60%; the resulting solution is possibly filtered, frozen, and that solvents are removed by sublimation under reduced pressure, at a low temperature, and thereafter the preparation is possibly distributed into doses in conditions assuring its sterility.

2. The method according to Claim 1, **characterised in that** the paclitaxel solution filtered through a sterilising filter is first dosed into containers in aseptic conditions and thereafter is frozen and solvents are removed by sublimation under reduced pressure, at a low temperature.

3. The method according to Claim 1 or 2 **characterised in that** solvent removal by sublimation is performed at the temperature in the range of-60°C to +50°C.

4. The method according to Claim 1 or 2 **characterised in that** the content of several solvents in the mixture is from 5% to 95% and the content of water is from 1% to 60%.

## Patentansprüche

1. Verfahren zum Erhalten einer stabilen pharmazeutischen Form von Paclitaxel als ein Antikrebsarzneistoff, **dadurch gekennzeichnet, dass** kristallines Paclitaxel in einem neutralen organischen Lösungsmittel, welches aus der Gruppe ausgewählt ist, die Acetonitril, Dioxan, Ethanol oder ein Gemisch von diesen Lösungsmitteln umfasst, gegebenenfalls mit einer Zugabe von Wasser gelöst wird, mit der Maßgabe, dass der Gehalt an mehreren Lösungsmitteln in dem Gemisch im Bereich von 5 % bis 95 % liegt, während der Gehalt an Wasser im Bereich von 0 bis 60 % liegt; die resultierende Lösung gegebenenfalls filtriert, gefroren wird und dass Lösungsmittel durch Sublimation unter verringertem Druck bei einer niedrigen Temperatur entfernt werden und danach die Zubereitung gegebenenfalls, unter Bedingungen, die ihre Sterilität sicherstellt, in Dosen aufgeteilt wird.

2. Verfahren gemäß Anspruch 1, **dadurch gekennzeichnet, dass** die Paclitaxel-Lösung, die durch einen Sterilfilter filtriert wurde, zuerst in Behälter unter aseptischen Bedingungen dosiert wird und danach gefroren wird und die Lösungsmittel durch Sublimation unter verringertem Druck bei einer niedrigen Temperatur entfernt werden.

3. Verfahren gemäß Anspruch 1 oder 2, **dadurch gekennzeichnet, dass** die Lösungsmittelentfernung bei der Temperatur im Bereich von -60°C bis +50°C durchgeführt wird.

4. Verfahren gemäß Anspruch 1 oder 2, **dadurch gekennzeichnet, dass** der Gehalt an mehreren Lösungsmitteln in dem Gemisch 5 % bis 95 % beträgt und der Gehalt an Wasser 1 % bis 60 % beträgt.

## Revendications

1. Procédé destiné à obtenir une forme pharmaceutique stable de paclitaxel en tant que médicament anticancéreux **caractérisé en ce que** du paclitaxel cristallin est dissous dans un solvant organique neutre choisi dans le groupe comprenant l'acétonitrile, le dioxane, l'éthanol ou un mélange de ces solvants, avec une addition possible d'eau, à condition que la teneur de chacun de ces solvants dans le mélange soit dans la gamme de 5 % à 95 %, alors que la teneur en eau est dans la gamme de 0 à 60 % la solution résultante est éventuellement filtrée, congelée, et ces solvants sont éliminés par sublimation sous pression réduite à une basse température, et après cela la préparation est éventuellement distribuée en des doses dans des conditions assurant sa stérilité.

2. Procédé selon la revendication 1, **caractérisé en ce que** la solution de paclitaxel filtrée à travers un filtre stérilisant est d'abord dosée dans des conteneurs dans des conditions aseptiques et est après cela congelée et les solvants sont éliminés par sublimation sous pression réduite, à une basse température.

3. Procédé selon la revendication 1 ou 2, **caractérisé en ce que** l'élimination des solvants par sublimation est effectuée à une température dans la gamme de -60 °C à + 50 °C.

4. Procédé selon la revendication 1 ou 2, **caractérisé en ce que** la teneur de chacun de ces solvants dans le mélange est de 5 % à 95 % et la teneur en eau est de 1 % à 60 %.
